# EUROPEAN PATENT APPLICATION

(11) **EP 2 082 714 A1**
(43) Date of publication of application: **29.07.2009**
(21) Application number: 08425026.5
(22) Date of filing: 17.01.2008
(51) Int. Cl.: A61F 13/15

(54) **Sanitary towel, in particular for female use**

(71) Applicant: Corman S.p.A., 20084 Lacchiarella MI (IT)
(72) Inventor: Mantovani, Giorgio, 20135 Milano (IT)
(74) Representative: Cinquantini, Bruno

(57) **Abstract**

A sanitary towel, in particular for female use, consisting of:
(a) an upper outer layer (2) made of absorbent cotton non-woven fabric, which is permeable to liquid;
(b) at least one inner absorbent layer (3, 4) made of long fibre absorbent cotton wool; and
(c) a lower outer layer (5) made of biodegradable and impermeable material.

Compared to traditional sanitary towels generally employing cotton, the sanitary towel of the invention results especially compatible with skin, i.e. it has no allergenic effects in combination with a high absorbency degree, in virtue of the kind of non-woven fabric of the layer in contact with skin, and in virtue of the kind of cotton wool of the intermediate or inner layer, all of which are obtained by a bleaching process by means of hydrogen peroxide.

## Description

The present invention relates to a sanitary towel, in particular a sanitary towel for female use.

Currently used sanitary towels consist of a multilayer pad, in which the absorbent portion is made of cellulose, which is impregnated with superabsorbent chemical powder, and is placed between two plastic material films.

The main drawback with these known structures is that the degree of absorbency required for the product is achieved by means of an artificial powder, the latter possibly inducing allergic reactions in sensitive individuals.

Another known embodiment features several layers which are generally made of cotton and are not suitable to lend the desired degree of liquid absorbency to the product. This kind of sanitary towel also displays problems as far as the compatibility with skin contact is concerned, also due to the presence of residues from chlorine bleaching.

It is the main object of the present invention to provide a sanitary towel, in particular for female use, which, in comparison to the known sanitary towels employing artificial material layers, does not induce allergic reactions, while maintaining or even improving its absorbency.

It is a further object of the invention to provide a sanitary towel of the above said type which offers a higher degree of absorbency, perspirability and softness in contact with skin in comparison to the known sanitary towels displaying cotton layers.

It is a yet another object of the invention to provide a sanitary towel which does not include allergenic materials resulting from the processing cycle thereof.

These and other objects are achieved by the sanitary towel according to claim 1. Preferred embodiments of the invention result from the remaining claims.

Compared to the known sanitary towels employing artificial or synthetic substances, the sanitary towel according to the invention offers the advantage that, the liquid absorbency being the same, it is totally compatible with skin, thus avoiding the occurrence of allergies.

Compared to traditional sanitary towels generally employing cotton, the sanitary towel of the invention results especially compatible with skin, i.e. it has no allergenic effects in combination with a high absorbency degree, in virtue of the kind of non-woven fabric of the layer in contact with the skin, and in virtue of the kind of cotton wool of the intermediate or inner layer, all of which are obtained by a bleaching process by means of hydrogen peroxide.

These and other objects, advantages and features result from the following description of a preferred embodiment of the sanitary towel of the invention disclosed by way of non-limitative example in the figures of the accompanying drawings.

Therein:
- figure 1 shows an overall perspective view of the sanitary towel of the invention; and

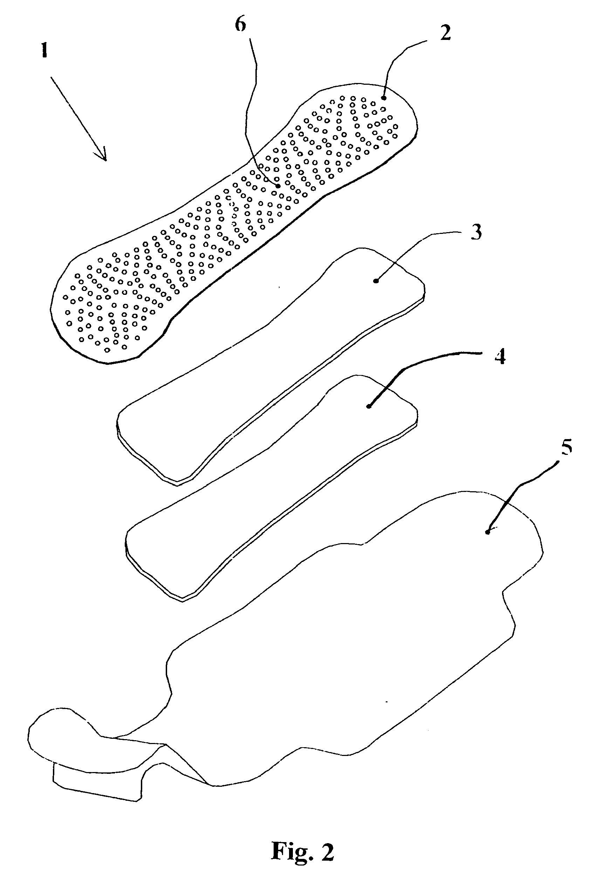

- figure 2 shows an exploded view of the sanitary towel in figure 1.

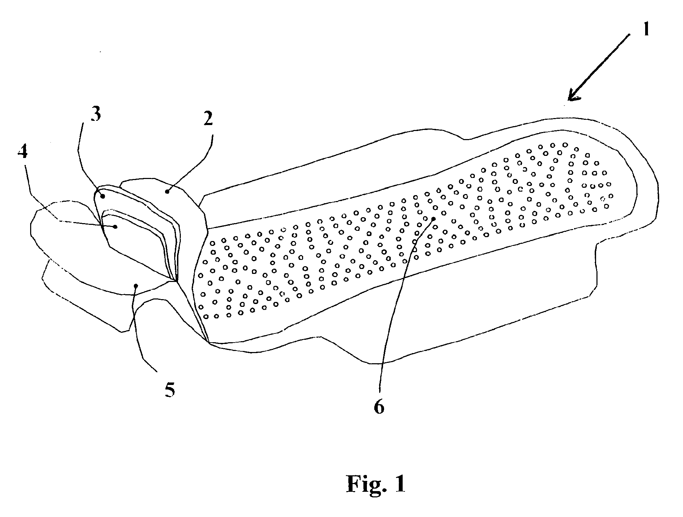

The sanitary towel of the invention is indicated as a whole with numeral 1 in figure 1 and is formed by the following layers of material, which are arranged in sequence:
- an upper outer layer 2 made of organic cotton wool non-woven fabric, which is permeable to liquid and is intended in use to come into contact with the skin;
- a first inner layer 3 made of long fibre organic cotton wool;
- optionally also a second inner layer 4, made of the same material forming the previous layer 3; and
- a lower outer layer 5 of natural and biodegradable impermeable material.

The material forming the upper outer layer 2 of the sanitary towel 1 of the invention, i.e. the layer intended to directly contact the skin of the user, is an organic cotton wool non-woven fabric, which is permeable to the organic liquids to be absorbed. In particular, it consists of cotton from organic agriculture, which is subjected to a hydrogen peroxide bleaching process and is interlaced with water.

The layer 2, which is formed in this manner, is also advantageously provided with a plurality of through-holes 6, which serve the function of promoting the permeability to liquid towards the innermost absorbent layers.

The type of non-woven fabric of the layer 2 of the sanitary towel of the invention leads the latter to be particularly stable and resistant, especially as far as the portion contacting the skin is concerned. Furthermore, the use of cotton prepared by hydrogen peroxide bleaching avoids the product from retaining the processing residues (usually chlorine), which may irritate the skin.

The material of the layer 2 specifically displays the following features;

**Table 1**

| **TEST** | **STANDARD** | **M.U.** | **SPECIFICATION** | |
|---|---|---|---|---|
| Weight | ISO 9073 1 | g/m² | 35 ± 10% | |
| Thickness | ISO 9073 2 | mm | 0,45 ± 0,10 | |
| Tensile strength | ISO 9073 3* | daN | L | T |
| | | | ≥2,0 | ≥2,0 |
| Wet tensile strength | ISO 9073.3* | daN | ≥1,5 | ≥1,5 |
| Elongation at fracture | ISO 9073.3* | % | ≤85 | ≤85 |
| Wet elongation at fracture | ISO 9073.3* | % | ≤85 | ≤85 |
| Absorbency | Edana 10.2.96/2 | % | >1000 | |
| Time of absorbency | Edana 10.2.96/1 | S | ≤3 | |
| Linting | Edana 300.0.84 | g/m² | ≤4,0 | |
| Acidity and alkalinity | Uni En 1644.1/F | pH | 6, 0÷8, 0 | |

| | | | | |
|---|---|---|---|---|
| * Clamp distance 100 mm | | | | |

The material forming the inner absorbent layers 3 and 4 consists of long fibre organic absorbent cotton wool having a high absorbency to liquids.

This material is as well obtained by hydrogen peroxide bleaching and by water interlacing, the latter being performed at a lower pressure than that employed in order to obtain the previous layer 2, so as to lend the material a soft and fluffy consistency which is peculiar to cotton wool.

The kind of material of the above described layers 3 and 4, i.e. long fibre absorbent cotton wool, increases the absorbency of the product, also lending it the highest degree of softness, so that it results especially comfortable to use.

The technical features of the material forming the layers 3 and 4 of the sanitary towel 1 are the following:

**Table 2**

| **TEST** | **STANDARD** | **M.U.** | **SPECIFICATION** | |
|---|---|---|---|---|
| Weight | ISO 9073 1 | g/m² | 120 ± 10% | |
| Thickness | ISO 9073 2 | mm | 1.1 + 0,10 - 0,15 | |
| Tensile strength | ISO 9073 3* | daN | **L** | **T** |
| | | | ≥2,5 | ≥2,5 |
| Wet tensile strength | ISO 9073.3* | daN | ≥2,0 | ≥2,0 |
| Elongation at fracture | ISO 9073.3* | % | ≤80 | ≤80 |
| Wet elongation at fracture | ISO 9073.3* | % | ≤100 | ≤100 |
| Absorbency | Edana 10.2.96/2 | % | ≥900 | |
| Time of absorbency | Edana 10.2.96/1 | S | ≤10 | |

| | | | | |
|---|---|---|---|---|
| * Clamp distance 100 mm | | | | |

The layer 5 of the sanitary towel 1 of the invention consists of a biodegradable and impermeable material film. Mater-Bi® from Novamont Spa-Novara is especially advantageous for the invention:

**Table 3**

| **PROPERTY** | **UNIT** | **TEST** | **AVERAGE** | **NOTES** |
|---|---|---|---|---|
| Melting temperature | °C | DSC | 110 | granules |
| Melt Flow Rate | g/10 min | ASTM D1238 | 3.5 | at T=150°C, weight=5Kg |
| Density | g/cm³ | Pycnometer | 1.27 | granules, at 23 °C |
| Tensile strength | Mpa | ASTM D882 | 25 | film thickness 30µm |
| Elongation at fracture | % | ASTM D882 | 300 | film thickness 30µm |
| Young's modulus | Mpa | ASTM D882 | 230 | film thickness 30µm |
| Tear resistance | N/mm | ASTM D1938 | 45 | i MD |
| | « | « | 45 | p MD |
| | « | « | 76 | i TD |
| | « | « | 76 | i PD |
| Haze | % | ASTM-D1003 | 89 | |
| Permeability to water vapour | gx30µm/m²x24h | ASTM E398 | 700 | film 30µm, 38°/90%RH |

The combination of the upper outer layer 2 made of absorbent cotton non-woven fabric with the inner layers 3 and 4 made of absorbent cotton wool, and with the lower outer layer 5 made of biodegradable material, lends the sanitary towel of the invention the properties of a natural product, which is suitable to prevent the irritation of skin, is comfortable when worn and has a high absorbency and perspirability.

The above described properties of the sanitary towel according to the invention are proved by the following tests:

### Perspirability test

Apparatus for measuring the permeability to water vapour: *Extrasolution PermeH₂O* model with a measuring cell with nitrogen flow (pure gas for analysis), at a flow rate of about 30 ml/min and a pressure in the range from 1.5 to 2.0 bars. Test surface: 2.27 cm². The apparatus is interfaced to a PC and the obtained data are processed by an application software.
- temperature: 37°± 0.1 °C
- relative humidity (permeating side): 100%
- minimum conditioning time: 15 minutes
- number of averaged points in a steady state: 500

The tests were performed in the intermediate areas of the aids.

Thickness determined by a Shirley apparatus (applied pressure: 0.1 Kpa, 23°C and 50% UR): 3.22 mm.

Perspirability value (*WVTr-Water Vapour Transmission rate* measured in the intermediate areas of the sanitary towel): 468.0 WVTr (g/m² 24h)

### Absorbency test

The test was performed by complete immersion of the sanitary towel in the test liquid. The sanitary towel was weighed dry at first and then wetted in the test liquid and weighed again in two different conditions:
- in wet conditions,
- after a certain compression.

All tests were performed according to the SSCCP (*Stazione Sperimentale Carta e Cartone e Paste per carta -* paper and cardboard and paper pulp experimental station, Milan) Method, where the absorbency is expressed as the amount of liquid that the sanitary towel is able to retain after being immersed for a predetermined time in the test liquid. All tests are performed with mimicking liquid in a conditioned environment at 23+1 °C with a relative humidity of 50 ± 2%.

The sanitary towels wetted in this manner are therefore pressed by rolling on a flat surface for at least 24h in ambient conditions. The results are summarised in the following table:

**Table 4 Absorbency, specific absorption and amount of test liquid retained after compression**

| Test n° | Weight of dry sanitary towel (g) | Weight of sanitary towel after immersion (g) | **Absorbency (g)** | **Specific absorption (g/g)** | Weight of sanitary towel after immersion (g) | **Amount of liquid retained after compression (g)** |
|---|---|---|---|---|---|---|
| I | 5,26 | 55,8 | 50,54 | 10,61 | 51,93 | 46,67 |
| II | 5,34 | 57,28 | 51,94 | 10,73 | 53,30 | 47,96 |
| III | 5,29 | 56,63 | 51,34 | 10,71 | 53,30 | 48,01 |
| IV | 5,32 | 57,09 | 51,77 | 10,73 | 52,78 | 47,46 |
| V | 5,3 | 57,52 | 52,22 | 10,85 | 52,88 | 47,58 |
| **Average value** | 5,30 | 56,9 | **51,6** | **10,7** | 52,84 | **47,54** |

| | | | | | | |
|---|---|---|---|---|---|---|
| Standard deviation | 0.03 | 0,7 | 0,65 | 0,09 | 0,56 | 0,54 |

## Claims

1. A sanitary towel, in particular for female use, **characterised in that** it consists of:
(a) an upper outer layer (2) made of absorbent cotton non-woven fabric, which is permeable to liquid;
(b) at least one inner absorbent layer (3, 4) made of long fibre absorbent cotton wool; and
(c) a lower outer layer (5) made of biodegradable and impermeable material.

2. A sanitary towel according to claim 1, **characterised in that** the cotton forming said layers (2, 3, 4) is an organic absorbent cotton, which is interlaced with water and obtained by a hydrogen peroxide bleaching process.

3. A sanitary towel according to claim 1 or 2, **characterised in that** the non-woven fabric forming said upper outer layer (2) of the sanitary towel (1) displays the following technical features:
**Table 1**
| **TEST** | **STANDARD** | **M.U.** | **SPECIFICATION** | |
|---|---|---|---|---|
| Weight | ISO 9073 1 | g/m² | 35 ± 10% | |
| Thickness | ISO 9073 2 | mm | 0,45 ± 0,10 | |
| Tensile strength | ISO 9073 3* | daN | L | T |
| | | | ≥2,0 | ≥2,0 |
| Wet tensile strength | ISO 9073.3* | daN | ≥1,5 | ≥1,5 |
| Elongation at fracture | ISO 9073.3* | % | ≤85 | ≤85 |
| Wet elongation at fracture | ISO 9073.3* | % | ≤85 | ≤85 |
| Absorbency | Edana 10.2.96/2 | % | >1000 | |
| Absorption time | Edana 10.2.96/1 | s | ≤3 | |
| Linting | Edana 300.0.84 | g/m² | ≤4,0 | |
| Acidity and alkalinity | Uni En 1644.1/F | pH | 6, 0÷8,0 | |
| | | | | |
|---|---|---|---|---|
| * Clamp distance 100 mm | | | | |

4. A sanitary towel according to claim 1 or 2, **characterised in that** said at least one inner absorbent layer (3, 4) consists of long fibre organic absorbent cotton wool having a high absorbency for liquids.

5. A sanitary towel according to claim 4, **characterised in that** the material forming said at least one layer (3, 4) has the following technical features:
**Table 2**
| **TEST** | **STANDARD** | **M.U.** | **SPECIFICATION** | |
|---|---|---|---|---|
| Weight | ISO 9073 1 | g/m² | 120 ± 10% | |
| Thickness | ISO 9073 2 | mm | 1.1 + 0,10 - 0,15 | |
| Tensile strength | ISO 9073 3* | daN | **L** | **T** |
| | | | ≥2,5 | ≥2,5 |
| Wet tensile strength | ISO 9073.3* | daN | ≥2,0 | ≥2,0 |
| Elongation at fracture | ISO 9073.3* | % | ≤80 | ≤80 |
| Wet elongation at fracture | ISO 9073.3* | % | ≤100 | ≤100 |
| Absorbency | Edana 10.2.96/2 | % | ≥900 | |
| Absorption time | Edana 10.2.96/1 | s | ≤10 | |
| | | | | |
|---|---|---|---|---|
| * Clamp distance 100 mm | | | | |

6. A sanitary towel according to claim 1 or 2, **characterised in that** said lower outer layer (5) consists of a film made of Mater-Bi® material from Novamont.
